# NEW EUROPEAN PATENT SPECIFICATION

(11) **EP 2 215 481 B2**
(45) Date of publication and mention of the opposition decision: **18.01.2017**
(45) Mention of the grant of the patent: 02.04.2014
(21) Application number: 08850368.5
(22) Date of filing: 14.11.2008
(51) Int. Cl.: G01N 33/68

(54) **DIAGNOSTIC USE OF INDIVIDUAL MOLECULAR FORMS OF A BIOMARKER**
DIAGNOSTISCHE VERWENDUNG INDIVIDUELLER MOLEKULARER FORMEN EINES BIOMARKERS
UTILISATION DIAGNOSTIQUE DE FORMES MOLÉCULAIRES PARTICULIÈRES D'UN BIOMARQUEUR

(30) Priority: 15.11.2007 US 988137 P
(43) Date of publication of application: 11.08.2010
(73) Proprietor: Bioporto Diagnostics A/S, 2900 Hellerup (DK)
(72) Inventor: UTTENTHAL, Lars, Otto, E-37002 Salamanca (ES); BANGERT, Kristian, DK-2840 Holte (DK)
(74) Representative: Høiberg A/S
(86) International application number: PCT/DK2008/050274
(87) International publication number: WO 2009/062520

(56) References cited:
- WO-A-2006/066587
- WO-A-2007/098102
- WO-A1-2009/052392
- WO-A2-2004/088276
- US- - 60/ 981 473
- KJELDSEN L ET AL: "Characterization of two ELISAs for NGAL, a newly described lipocalin in human neutrophils" JOURNAL OF IMMUNOLOGICAL METHODS, ELSEVIER SCIENCE PUBLISHERS B.V.,AMSTERDAM, NL, vol. 198, no. 2, 13 November 1996 (1996-11-13), pages 155-164, XP004071802 ISSN: 0022-1759
- RUDD P M ET AL: "Glycosylation of natural human neutrophil gelatinase B and neutrophil gelatinase B-associated lipocalin" BIOCHEMISTRY 19991019 AMERICAN CHEMICAL SOCIETY US,, vol. 38, no. 42, 19 October 1999 (1999-10-19), pages 13937-13950, XP002508176
- MISHRA J ET AL: "Identification of neutrophil gelatinase-associated lipocalin as a novel early urinary biomarker for ischemic renal injury" JOURNAL OF THE AMERICAN SOCIETY OF NEPHROLOGY, WILLIAMS AND WILKINS, BALTIMORE, MD, US, vol. 14, no. 10, 1 October 2003 (2003-10-01), pages 2534-2543, XP002377943 ISSN: 1046-6673
- Product specification, Mouse monoclonal antibody, biotinylated
- Mouse monoclonal antibody
- Human monomer NGAL Matched Antibody Pair
- Product specification; Human NGAL monomer-specific ELISA Kit
- Transscipts from Suzhou company register with English translations
- Letter Assignment
- Letter Assignment rec.
- Letter - Transcript from Danish company register
- Recordation of assignments for V3a
- Data sheet for V3
- Recordation of assignments for two other priority applications for V3.
- MISHRA J. ET AL: 'Neutrophil gelatinase-associated lipocalin (NGAL) as a biomarker for acute renalinjury after cardiac surgery' WWW.THELANCET.COM vol. 365, 02 April 2005, pages 1231 - 1238
- NICKOLAS T.L. ET AL: 'Sensitivity and Specificity of a Single Emergency Department Measurement of Urinary Neutrophil Gelatinase-Associated Lipocalin for Diagnosing Acute Kidney Injury' ANN INTERN MED. vol. 148, no. 11, 03 June 2008, pages 810 - 819
- Medical Subject (MeSH): "Acute Kidney Injury", National Center for BiotechnologyInformation, U.S. National Library of Medicine, 8600 Rockville Pike, Bethesda MD,20894 USA; downloaded from the Internet on 30 December 2014
- Application Data Sheet for V3a
- J. A. HANLEY, PH. D. ET AL.: 'The Meaning and Use of the Area under a Receiver Operating Characteristic (ROC) Curve¹' RADIOLOGY vol. 143, no. 1, April 1982, pages 29 - 36
- J. A. HANLEY, PH. D. ET AL.: 'A Method of Comparing the Areas under Receiver Operating Characteristic Curves Derived from the Same Cases¹' RADIOLOGY vol. 148, no. 3, September 1983, pages 839 - 843
- Binding characteristics of ABS 062-33 and ABS 062-14
- Biostatistical comments on patent EP2215481
- Certificate of approval

## Description

### Field of the invention

The present invention relates to the diagnosis and monitoring of pathologic conditions characterized by an alteration in levels and molecular forms of neutrophil gelatinase-associated lipocalin (NGAL), in particular a recent or ongoing injurious or pathological process in a mammal that has affected or is affecting the kidneys and has led or may lead to renal injury or acute renal failure (ARF). As such it is relevant to internal medicine and in particular to critical or intensive care medicine, including traumatology, and to surgery, oncology and diagnostic imaging, where procedures that may injure the kidneys are carried out. It is also relevant to veterinary medicine and surgery and to the study of renal pathophysiology and noxious or therapeutic influences on the kidney in laboratory animals. The scope of protection is defined by the claims.

### Background of the invention

The present invention relates to the diagnostic use of a new protein biomarker of renal pathology that appears in both blood and urine, to detect, monitor and assess the severity of not only renal disorder or injury in a human being or mammal but also disorder or injury of other organs, tissues or cell types that release this protein. The protein biomarker is neutrophil gelatinase-associated lipocalin (NGAL), also known as neutrophil lipocalin (NL; HNL in the case of human neutrophil lipocalin), lipocalin 2 (LCN2) or siderocalin in various animals including humans, 25-kDa alpha 2-microglobulin-related protein or neu-related lipocalin (NRL)in the rat, or 24P3, 24-kDa secreted inducible protein (SIP24) or uterocalin in the mouse. Human NGAL is a glycoprotein that was first isolated from the granules of neutrophil polymorphonuclear leukocytes, hereinafter referred to as neutrophils (Allen et al., 1989; Venge et al., 1990, Triebel et al., 1992; Kjeldsen et al., 1993). The mature protein consists of a single protein chain of 178 amino acid residues and its molecular mass is variously estimated as 24 or 25 kDa in its glycosylated state. The protein contains an intrachain disulfide bridge and the human form contains an additional cysteinyl residue that is thought to participate in the formation of complexes with itself or with neutrophil gelatinase. This additional cysteinyl residue is not present in the NGAL of the non-human species for which cDNA sequences are available to date (chimpanzee, Rhesus monkey, dog, pig, horse, rat and mouse). NGAL isolated from neutrophils regularly contains a substantial proportion of homodimers and occasionally material of higher molecular mass that has been postulated to be a homotrimer. In addition, traces of higher homooligomers of NGAL of molecular mass varying from about 150 kDa to over 200 kDa have been detected. The fact that NGAL isolated or secreted from human neutrophils *in vitro* contains a high proportion of homodimers has led its Molecular mass to be reported as 40 or 45 kDa (Venge et al., 1990; Xu et al., 1994).

A small and variable proportion of the total NGAL isolated or secreted from human neutrophils *in vitro* is associated with 92-kDa human neutrophil gelatinase, also called gelatinase B, type IV collagenase or matrix metalloproteinase 9 (MMP-9), either as an NGAL monomer forming a complex of apparent kDa 115 (Monier et al., 2000; Yan et al., 2001) or as an NGAL dimer, forming a complex of apparent kDa 125 (Yan et al., 2001). These complexes have also been identified in the urine of patients with a variety of cancers, including cancers of the prostate, bladder, kidney and breast.

NGAL homodimers, homotrimers, higher homooligomers and NGAUMMP-9 complexes can be dissociated into their constituent monomers by reduction, suggesting that the complexes are, at least in part, covalently linked by disulfide bridges. However, the presence in human NGAL of a single cysteinyl residue which does not participate in the intrachain disulfide bridge cannot explain the formation of disulfide-linked trimers or higher oligomers, whether these consist of NGAL alone or of NGAL in complex with MMP-9. The mechanisms by which these postulated homotrimers, higher oligomers, or complexes between MMP-9 and NGAL homodimer are held together remain to be elucidated. It has been possible to generate NGAL/MMP-9 complexes *in vitro* by co-incubating recombinant human NGAL monomer with recombinant human MMP-9 in the presence of buffer containing calcium and zinc ions (Yan et al., 2001). These complexes are reported as having a molecular mass of 115 kDa and to have a transient existence, whereas the most common form of complex seen in the urine of patients with cancer is the 125-kDa form.

Allen et al. (1989) also observed that the apparent molecular mass of NGAL from human neutrophils increased from 24 kDa to 50-70 kDa in the presence of calcium ions, suggesting the formation of dimers and trimers which, in the opinion of the present inventors, might be non-covalently linked, at least initially.

It is thus apparent that human NGAL occurs in neutrophils in multiple molecular forms, including monomers, homodimers (as a major component), homotrimers, higher homooligomers, monomer complexed with MMP-9 and homodimer complexed with MMP-9. In addition, NGAUMMP-9 complexes of even higher apparent molecular mass, in the order of 200 kDa, have been observed. These may contain MMP-9 homodimers and an undetermined number of NGAL molecules. The individual subunits of these molecular forms are at least in part held together by interchain disulfide bridges, although the cysteinyl residues that participate in these in the different complexes have not been identified with certainty. It is also possible that non-covalent forces participate in complex formation.

Less information is available on the multiple molecular forms of NGAL in non-human mammals such as the rat and mouse. However, Bu et al. (2006) have shown that NGAL and MMP-9 are co-synthesized by rat vascular smooth muscle cells in response to angioplastic injury or stimulation with interleukin-1β. The rat NGAL was shown by Western blotting to occur in monomer, dimer and trimer forms, as well as a 150-kDa form. MMP-9 occurred in monomer and dimer forms. While complexes of rat NGAL and MMP-9 could not be demonstrated in lysed rat intimal smooth muscle cells, NGAUMMP-9 complexes of approximately 115 and 125 kDa were demonstrated in the culture medium conditioned by these cells, demonstrating the extracellular but not intracellular formation of NGAUMMP-9 complexes. The protein chain of rat NGAL contains only two cysteinyl residues, which are presumed to form an intrachain disulfide bridge. However, the non-availability of an additional cysteinyl residue in the protein chain does not seem to interfere with the formation of multiple molecular forms of NGAL in the rat, which behave on Western blotting in the unreduced and reduced state as if they depend on the presence of interchain disulfide links. It is unknown how the subunits of multiple molecular forms observed in the rat are linked. One possibility is that disulfide interchange occurs to break the intrachain disulfide bridge so that interchain disulfide linkages may form.

Zhao et al. (2006) have also demonstrated the presence of NGAUMMP-9 complex in the plasma of both wild-type and RECS-1 knockout mice which are prone to aortic dilation. Like rat NGAL, the mouse NGAL protein chain contains only two cysteinyl residues.

Thus, despite the absence of a third cysteinyl residue in the NGAL protein chains from the non-human mammals analyzed so far, the evidence from the rat and mouse suggests that NGAL in non-human mammals occurs in multiple molecular forms that are comparable with those observed in man.

As a diagnostic biomarker, human NGAL was initially disclosed (U.S. Patent 6,136,526; PCT application WO95/29404), under its denomination HNL, as a specific marker of neutrophil activation, being released into the blood when invading microorganisms, in particular pyogenic bacteria, cause degranulation of the neutrophils and exocytosis of the granule proteins. As such, the measurement of elevated levels of NGAL in a plasma or serum sample from a human was proposed to indicate that the individual is suffering from an inflammatory process, especially one caused by a bacterial infection. This document discloses that immunoblotting of non-reduced material released *in vitro* from neutrophils contained three HNL-immunoreactive bands with apparent molecular weights of >200 kDa, 45 kDa and 24 kDa. After reduction of the released material only one band at an apparent molecular weight of 24 kDa was seen. No disclosure was made with respect to the concurrent existence of different molecular forms of the protein in the samples of bodily fluids on which the measurement of HNL was performed, nor of the use of any quantification method that might be selective or specific for one of the molecular forms disclosed.

In the mouse, NGAL (under its denomination SIP24 or 24P3) was identified as an acute phase protein of type 1, its expression being mainly located in the liver during the acute phase response (Liu and Nilsen-Hamilton, 1995). This document does not disclose the occurrence of multiple molecular forms of the protein. It also contradicts, in another species, the assertion made in U.S. Patent 6,136,526 that NGAL is a protein that is completely specific to neutrophil granulocytes.

U.S. Patent Application 2004/0219603 discloses the use of NGAL as a urinary biomarker for detecting renal tubular cell injury at an early stage. U.S. Patent Application 2005/0272101 discloses the use of NGAL in blood serum for the same purpose. However, neither disclosure describes how renal tubular cell injury can be discriminated from systemic inflammation, or bacterial infection, or cancers as the cause of the elevated NGAL level. Neither document discloses the existence of multiple molecular forms of NGAL, nor the use of any selective or specific measurement of a particular molecular form of NGAL.

The present inventors have previously filed a PCT application WO2006/066587 which discloses how NGAL levels in urine or blood plasma or serum can be separated by means of a cutoff level into lower elevations that are not diagnostic of renal injury leading to ARF and higher elevations that are. WO2006/066587 takes into account the fact that NGAL levels in bodily fluids may also be elevated in inflammations, infections and certain cancers, but usually to lower values than those associated with renal injury leading to ARF.

The present inventors have also filed a U.S. Provisional Patent Application (No. 60/919,277) which discloses a method of distinguishing between rises of NGAL in bodily fluids that are due to renal injury and those that are due to non-renal causes, without resorting to the use of a cutoff value for the concentration of NGAL in a given bodily fluid. This method comprises determining the ratio of the NGAL concentration in a sample of urine to that present at the same time in a sample of blood plasma or serum. A ratio exceeding a value approximating to unity indicates that the individual from whom the samples were taken has suffered a renal injury that has led to ARF or signifies an immediate risk of the individual developing ARF.

Neither of the aforesaid disclosures by the present inventors depends on the detection or measurement of a particular molecular form of NGAL, just as none of the above-mentioned disclosures by other authors relating to the diagnostic use of NGAL determination discloses the measurement of a specific individual molecular form of NGAL that is not complexed with MMP-9.

### Definitions

The present disclosure makes use of the following terms which, because they have sometimes been used with various specialized or restrictive meanings in prior art, require definition of the sense in which they are used herein.

**Renal disorder:** Any abnormal or pathological condition which affects the functioning of the kidney, whether the condition arises within the kidney or outside it.

**Renal disease:** Any renal disorder considered to constitute a classifiable disease entity.

**Renal injury:** Any injury to the kidney, be it physical (e.g. traumatic), chemical (nephrotoxic) or pathological (disease related) in origin, in particular any injury which affects a sufficient number of renal cells to produce a detectable impairment of a renal function.

**Acute renal injury:** A renal injury that arises rapidly, typically within a few hours.

**Acute renal failure (ARF):** A rapidly arising, variable (i.e. not necessarily complete) impairment or loss of the main excretory and homeostatic functions of the kidney, as typically evidenced by a rise in serum creatinine over and above the fluctuations due to metabolic changes and/or by a fall in the rate of urine output.

### Summary of the invention

By analyzing samples of bodily fluids from patients who showed clinical and biochemical evidence of renal disorders including acute renal injury, and from control individuals who did not, we have surprisingly found that the NGAL released into the blood and urine by the affected kidneys is largely in the free monomer form. This finding contrasts with the immunoreactive NGAL that we have found at lower levels in the blood serum of normal individuals, which is thought to derive mostly from non-renal sources such as circulating neutrophils and maybe certain epithelial cells, and consists of higher NGAL complexes including NGAL/MMP-9 complexes, NGAL homotrimer, NGAL homodimer, and NGAL monomer. It also contrasts with the NGAL found in bodily fluids in other pathologic conditions, such as certain adenocarcinomas, which is characterized by the presence of NGAUMMP-9 complexes and NGAL oligomers in addition to NGAL monomer.

This observation makes it possible to improve the specificity of NGAL measurement in blood plasma, whether separated from anticoagulated whole blood prior to measurement or filtered from anticoagulated whole blood as part of the measurement procedure, or in blood serum or in urine, to diagnose renal disorders including renal injury. It is evident that the same principle of measuring individual molecular forms of NGAL can be applied to the diagnosis of disorders or injuries of other organs, tissues and cell types that release a different pattern of these molecular forms than the kidney.

Accordingly the present invention relates to a method of diagnosing, monitoring or assessing the severity of disease or injury to an organ, tissue or cell type in a mammal by measuring the concentration of an individual molecular form of neutrophil gelatinase-associated lipocalin (NGAL) in a sample of bodily fluid from the mammal. In a particular embodiment the mammal is a human individual. In a particular embodiment the individual molecular form of NGAL is free NGAL monomer.

The free NGAL monomer may be measured by means of a molecule that binds specifically to free NGAL monomer and not to complexed forms of NGAL of the mammalian species to which the method is applied. The free NGAL monomer may also be measured by means of an NGAL-binding molecule or combination of such molecules that bind to both free NGAL monomer and complexed forms of NGAL, once the free NGAL monomer has been separated from the complexed forms by a physical or chemical method that is both rapid and suitable for automation, these requirements excluding gel electrophoresis. The free NGAL monomer may also be measured indirectly from the difference between total and complexed NGAL in a sample, total NGAL being measured by means of an NGAL-binding molecule or combination of such molecules that bind to both free NGAL monomer and complexed forms of NGAL, and complexed NGAL being measured by means of a combination of NGAL-binding molecules that are incapable of binding at one and the same time to free NGAL monomer.

In another example the individual molecular form of NGAL is NGAL homodimer.

In another example, a combination of NGAL homodimer and higher oligomers, but excluding free NGAL monomer, is measured. This combination of molecular forms of NGAL may be measured by a combination of binding molecules that are incapable of binding at one and the same time to free NGAL monomer.

A further aspect of the present invention is the use of an individual molecular form of NGAL as a diagnostic marker molecule for disease, or cellular or tissue injury in a mammal, in which said molecular form of NGAL is NGAL which has been glycosylated in a particular manner by its cells of origin, that particular manner being distinguishable from NGAL that has been glycosylated in another way by different cells of origin.

One aspect of the present invention comprises using methods of measuring NGAL in bodily fluids that are specific or selective for the free monomer form of NGAL. These methods will record lower levels of NGAL than non-selective methods of NGAL measurement in normal individuals or patients with elevations of NGAL levels due to other causes than renal injury or disorder. At the same time, the elevation of NGAL monomer due to renal injury or disorder will be fully read by the monomer-specific measurement method. The use of the monomer-specific NGAL measurement method will therefore be more selective for detecting renal injury or disorder than those methods of measuring NGAL that show little or no selectivity for its different molecular forms.

Conversely, methods which measure exclusively homodimers or methods which measure exclusively homodimers and higher oligomers of NGAL may be useful for the diagnosis of conditions characterized by elevated levels of NGAL secreted by non-renal tissues that synthesize these molecular forms of NGAL.

Accordingly, a further aspect relates to diagnosing, monitoring or assessing the severity of disease or injury to an organ, tissue or cell type in a mammal, said disease or injury being characterized by a certain pattern of individual molecular forms of NGAL being present in a bodily fluid from said mammal, by measuring the concentrations of two or more individual molecular forms of NGAL in a sample of the bodily fluid.

The measurement of NGAUMMP-9 complexes is known to prior art and forms no part of the present invention, except in so far as said measurement be made in order to compare the result with results obtained according to the present invention. The measurement of NGAUMMP-9 complex is described, for example, in the product inlay of the Quantikine Human MMP-9/NGAL Complex Immunoassay kit, Catalog Number DM9L20, R&D Systems, Inc., Minneapolis, MN, USA.

Specific ligand binding methods have been devised which specifically measure free NGAL monomers or homodimers and higher oligomers of NGAL, respectively.

### Description of the drawings

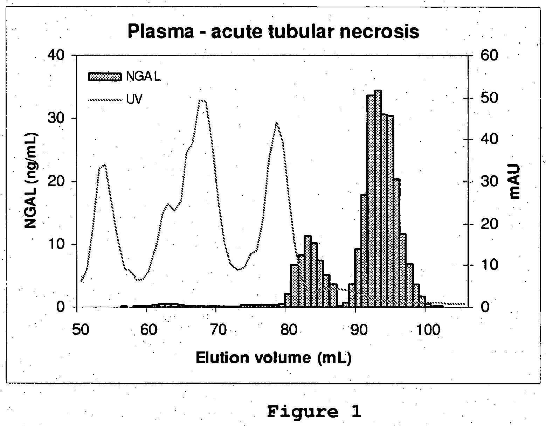

**Figure 1**
   Molecular size exclusion chromatography on a column Superdex 200 (GE Healthcare) of plasma from a patient with acute tubular necrosis. The line shows peaks of UV absorption at 280 nm corresponding to (left to right) macroglobulins (peak at approximately 54 mL), immunoglobulins (peak at approximately 69 mL) and albumin (peak at approximately 79 mL). The blocked bars show NGAL immunoreactivity in the collected fractions analyzed with an ELISA which detects both monomer and oligomerized forms of NGAL. Four regions of NGAL immunoreactivity were observed: a small peak emerging at approximately 60-65 mL and attributable to higher complexed forms of NGAL (1.0% of total NGAL immunoreactivity assignable to an individual molecular form), a trace amount emerging at approximately 74-79 mL and attributable to NGAL homotrimer (0.8% of total assignable NGAL immunoreactivity), a larger peak emerging at approximately 79-87 mL and attributable to NGAL homodimer (22.3% of total assignable NGAL immunoreactivity), and a large peak emerging at approximately 88-101 mL and attributable to free NGAL monomer (75.9% of total assignable NGAL immunoreactivity).
**Figure 2**
   Molecular size exclusion chromatography of urine from another patient with acute tubular necrosis performed as in Figure 1. Two peaks of NGAL immunoreactivity were observed: a small peak emerging at approximately 78-86 mL and attributable to NGAL homodimer (3.0% of total assignable NGAL immunoreactivity), and a large peak emerging at approximately 87-99 mL and attributable to free NGAL monomer (97.0% of total assignable NGAL immunoreactivity).
**Figure 3**
   Molecular size exclusion chromatography of normal human serum performed as in Figure 1. Four peaks of NGAL immunoreactivity were observed: a peak emerging at approximately 56-65 mL and attributable to higher complexed forms of NGAL (24.1% of total assignable NGAL immunoreactivity), a peak emerging at approximately 70-78 mL and attributable to NGAL homotrimer (24.5% of total assignable immunoreactivity), a peak emerging at approximately 78-86 mL and attributable to NGAL homodimer, (33.1% of total assignable NGAL immunoreactivity), and a peak emerging at approximately 88-100 mL and attributable to free NGAL monomer (18.3% of total assignable NGAL immunoreactivity).
**Figure 4**
   Receiver operating characteristic (ROC) plots for plasma levels of NGAL monomer (crosses), NGAL homodimer (rhomboids) and "total" NGAL (triangles), "total" NGAL comprising monomer and homodimer forms with a possible contribution from higher oligomeric forms. Peak plasma levels were obtained for 38 patients admitted to intensive care, 19 of which were diagnosed as having acute kidney injury. The plasma levels of NGAL homodimer are seen to be unsuitable for the diagnosis of acute kidney injury.

### Detailed Description of the Invention

In a series of critically ill patients admitted to intensive care, we have observed that the rise in body fluid concentrations of NGAL due to renal disorder or injury gives rise to a different pattern of molecular forms of NGAL in the blood plasma and urine than the pattern normally observed in healthy individuals or in patients without renal disorder or injury.

In a healthy individual, NGAL immunoreactivity in the blood plasma or serum occurs in molecular forms (estimated concentration ranges in brackets) corresponding to the free NGAL monomer (10-50%), a generally larger amount of NGAL homodimer (25-60%), and smaller amounts of NGAL homotrimer (5-35%) and higher complexed forms of NGAL including NGAUMMP-9 complex (5-35%). In the urine of healthy individuals, the basal level of total NGAL immunoreactivity is very low and for the time being it has been impracticable to analyze the relative content of the aforementioned molecular forms.

In patients with a moderately elevated level of NGAL due to non-renal disease, the pattern of NGAL molecular forms in the blood plasma remains essentially similar to that seen in the normal basal state. In these patients the pattern of NGAL molecular forms in urine is such that the monomer form predominates, but there is still a moderate amount of NGAL homodimer present. The presence of NGAL/MMP-9 complex in urine is irregular.

In patients with elevated levels of NGAL due to acute renal injury, the pattern of NGAL molecular forms in the blood plasma is very different from that seen in the normal state or in non-renal disease. The predominant molecular form in blood plasma is free NGAL monomer (70-80%), with a relatively small amount of NGAL homodimer (20-30%), a trace of NGAL homotrimer (0-2%), and a slight and irregular presence of higher molecular size forms including NGAL/MMP-9 complexes (0-2%).

These observations suggest that the predominant molecular form of NGAL secreted by the kidney in response to renal injury is the free monomer form of NGAL. Only a relatively small amount of homodimer is secreted, and secretion of higher molecular size forms of NGAL including NGAL/MMP-9 complexes is slight and does not regularly occur.

Hitherto, NGAL has been proposed as a marker molecule for renal disorders including acute renal injury without specifying that a particular molecular form of NGAL is involved. We now propose that the molecular marker of renal injury is NGAL monomer, as neutrophils and other cell types that also release NGAL to their surroundings typically also release large amounts of NGAL homodimer, and varying amounts of higher oligomers of NGAL including NGAL/MMP-9 complex.

In consequence, we propose that renal injury can be diagnosed with greater diagnostic specificity if the concentration of the free NGAL monomer is measured by a method specific for that molecular form, instead of measuring the mixture of different molecular forms of NGAL by existing methods that are not known to be selective for any of these forms. With respect to the diagnostic applicability of the method in view of the rapid progression of acute renal injury, it is desirable that the method of measurement should be rapid, so that the result is available within a short time, such as within 4 hours or less. This would preclude the separation of molecular forms of NGAL by gel electrophoresis, which has been used in prior art for the retrospective analysis of molecular forms of NGAL.

Accordingly, the present disclosure comprises a method of diagnosing or monitoring a renal injury, disease or disorder which has led to ARF or which signifies an immediate risk of developing ARF in a mammal, said method comprising the steps of
i) determining the concentration of free NGAL monomer in a sample of bodily fluid from the mammal and
ii) comparing said concentration value with a cutoff value determined from the range of free NGAL monomer concentrations in other individuals of that mammalian species which show no evidence of renal injury, disease or disorder, so that a value greater than the cutoff value indicates the presence of said renal injury, disease or disorder.

In a preferred example step i) of the method disclosed above does not include the use of gel electrophoresis.

In particular examples of the method, the cutoff value for samples of blood plasma or blood serum may be a value between 60 ng/mL and 600 ng/mL, such as 65 ng/mL or more, such as 70 ng/mL or more, such as 80 ng/mL or more, such as 90 ng/mL or more, such as 100 ng/mL or more, such as 120 ng/mL or more, such as 150 ng/mL or more, such as 200 ng/mL or more, such as 250 ng/mL or more, such as 300 ng/mL or more, such as 350 ng/mL or more, such as 400 ng/mL or more, such as 450 ng/mL or more, such as 500 ng/mL or more, such as 550 ng/mL or more; and the cutoff value for samples of urine may be a value between 10 ng/mL and 600 ng/mL, such as 15 ng/mL or more, such as 20 ng/mL or more, such as 30 ng/mL or more, such as 40 ng/mL or more, such as 50 ng/mL or more, such as 60 ng/mL or more, such as 70 ng/mL or more, such as 80 ng/mL or more, such as 90 ng/mL or more, such as 100 ng/mL or more, such as 120 ng/mL or more, such as 150 ng/mL or more, such as 200 ng/mL or more, such as 250 ng/mL or more, such as 300 ng/mL or more, such as 350 ng/mL or more, such as 400 ng/mL or more, such as 450 ng/mL or more, such as 500 ng/mL or more and such as 550 ng/mL or more.

Further, the present invention relates to a method of diagnosing, monitoring or determining the risk of developing acute renal failure in a mammal, wherein said method discriminates between an individual which does not have acute renal failure and is not at immediate risk of developing acute renal failure, and an individual which may have acute renal failure or is at risk of developing acute renal failure, said method comprising the steps of
i) determining the concentration of free NGAL monomer in a sample of bodily fluid from the mammal and
ii) comparing said concentration with a predetermined cutoff value chosen so that a concentration of free NGAL monomer below the cutoff value categorizes the mammal as not having and not being at immediate risk of developing acute renal failure.

In a preferred example step i) of the method disclosed above does not include the use of gel electrophoresis. In particular examples of the method, the cutoff value for samples of blood plasma or blood serum may be a value between 600 ng/mL and 60 ng/mL, such as 550 ng/mL or less, such as 500 ng/mL or less, such as 450 ng/mL or less, such as 400 ng/mL or less, such as 350 ng/mL or less, such as 300 ng/mL or less, such as 250 ng/mL or less, such as 200 ng/mL or less, such as 150 ng/mL or less, such as 120 ng/mL or less, such as 100 ng/mL or less, such as 90 ng/mL or less, such as 80 ng/mL or less, such as 70 ng/mL or less, such as 65 ng/mL or less; and the cutoff value for samples of urine may be a value between 600 ng/mL and 10 ng/mL, such as 550 ng/mL or less, such as 500 ng/mL or less, such as 450 ng/mL or less, such as 400 ng/mL or less, such as 350 ng/mL or less, such as 300 ng/mL or less, such as 250 ng/mL or less, such as 200 ng/mL or less, such as 150 ng/mL or less, such as 120 ng/mL or less, such as 100 ng/mL or less, such as 90 ng/mL or less, such as 80 ng/mL or less, such as 70 ng/mL or less, such as 60 ng/mL or less, such as 50 ng/mL or less, such as 40 ng/mL or less, such as 30 ng/mL or less, such as 20 ng/mL or less and such as 15 ng/mL or less.

In a particular embodiment of the present invention the monitoring methods above comprise the further step of repeating steps i) and ii) one or more times, particularly during the course of critical illness, after a medical or surgical procedure known to entail a risk of renal injury, or after a treatment of acute renal failure has been initiated or completed.

Said steps i) and ii) may in a particular embodiment be repeated within 24 hours, e.g. within 12 hours, such as within 6 hours, e.g. within 3 hours, such as within 1 hour, e.g. within 30 minutes or within 15 minutes.

Practice of the method requires a measurement method that is specific for free NGAL monomers. In a preferred embodiment of the invention, the measurement method is a ligand binding assay such as an immunochemical assay, using specific monoclonal antibodies or other specific binding molecules, however generated, to specifically bind to and measure the free monomer form of NGAL, while not measuring the other molecular forms of NGAL. Details of such a method are given in **Example 4** below.

Conversely, in another example, it is also possible to measure the concentration of NGAL homodimer in a sample of bodily fluid from a mammal. Details of such a method are given in **Example 5** below. The measurement of NGAL homodimers may prove to be of use in diagnosing affections of other organs or tissues that release NGAL homodimers, such as neutrophils and a range of non-renal epithelia, including epithelia of the respiratory tract, the gastrointestinal tract including the liver, and the urogenital tract including the mammary gland. The measurement of NGAL homodimers may be relevant to the detection of inflammation, whether systemic or localized, and the diagnosis of pathologic conditions affecting cells, tissues or organs of said tracts, or indeed any cell type, tissue or organ found to secrete NGAL homodimers.

It is possible to determine the concentrations of both free NGAL monomer on the one hand and NGAL homodimer plus higher oligomers on the other hand in a sample of bodily fluid by means of a combined assay that gives both results in a rapid and convenient manner without requiring the separation of the two molecular forms. This is described in **Example 6** below. Free NGAL monomer, NGAL homodimer and higher molecular size forms of NGAL can also be analyzed in a sample by separating these forms by a protein separation method, after which the amount of each molecular form is determined by one of the non-specific methods of measuring NGAL known to prior art. Combining the measurement of free NGAL monomer and NGAL homodimer plus higher oligomers by a non-separating method with established methods of determining NGAL/MMP-9 complex will give a reasonably complete profile of the molecular forms of NGAL present in the sample, without the need for protein separation, e.g. by chromatographic analysis. This profile of NGAL molecular forms may be of diagnostic use to identify affection of a particular organ or concurrent diseases affecting different organs.

One aspect of analyzing the profile of NGAL molecular forms comprises determining the absolute concentrations of NGAL monomer and homodimer as stated above and calculating the ratio between them. The ratio obtained in a sample from a given individual can be used to distinguish between pathologies characterized by determined ranges of values of the NGAL monomer:homodimer ratio. For example, the ratio of NGAL monomer to homodimer observed in plasma or serum in acute renal injury is above unity, such as a value of 1.5 or more, such as 2 or more, such as 3 or more, such as 4 or more, such as 5 or more, and up to 10 or more. The ratio of NGAL monomer to homodimer observed in urine in acute renal injury is also well above unity and typically greater than in plasma, comprising any value between 2 and 100 or more, such as 3 or more, such as 4 or more, such as 5 or more, such as 7 or more, such as 10 or more, such as 20 or more, such as 30 or more and such as 50 or more.

A further method of analyzing a profile of NGAL molecular forms is to determine the concentration of one or more of those forms of NGAL which are not complexed with MMP-9 by the methods here disclosed and comparing this to the concentration of NGAL/MMP-9 complexes determined by other methods. The ratio between the concentration of those forms of NGAL which are not complexed with MMP-9 and the concentration of NGAUMMP-9 complexes may be calculated. In plasma, serum or urine these ratios may range from values below unity to any value above unity in different pathologic conditions. Low ratios (e.g. from below unity to a value of 5) are expected in certain neoplastic conditions, and higher ratios (e.g. above 5) are expected in non-neoplastic disorders. Indeed, in many cases no NGAL/MMP-9 complex is detected.

The general principle of the present disclosure is to render the use of NGAL determination diagnostically specific to affections of different organs and tissues by exploiting the specific differences in the molecular forms of NGAL produced by its different cells of origin in response to disease or harmful stimuli. A further aspect of the present disclosure is that differences in the glycosylation of NGAL by different types of cells or tissues can also be exploited diagnostically to measure NGAL originating from a particular tissue or organ.

The methods of the present invention are particularly useful in patients admitted to intensive or critical care departments and will also be useful in patients who, while not being critically ill, have suffered a well-defined insult such as a surgical operation that may lead to ischemic injury of the kidney or have been exposed to nephrotoxic agents such as intravenously administered contrast media for diagnostic imaging, or have been exposed to nephrotoxic chemotherapeutic agents. The methods of the present invention are also particularly useful for determining the risk of developing ARF due to renal injury caused by ischemia, or due to a complication of an inflammatory, infective or neoplastic disease, or any cause requiring intensive care, or a surgical intervention, in particular where the surgical intervention may lead to ischemic injury of the kidney. In these cases the early identification of patients whose kidneys have been affected by a disease process or a medical or surgical procedure and who are therefore at risk of ARF may allow for an earlier and more intensive intervention to prevent the development of ARF. The methods of the present invention are also useful for diagnosing and monitoring disorders or injuries of other organs, tissues and cell types that release a different pattern of molecular forms of NGAL than that released by the kidney. In addition, said methods will be useful for determining organ and tissue damage due to injury from external physical or chemical causes or from exposure to radiation.

The methods of the present invention will also be useful in veterinary medicine, the investigation of renal pathophysiology in experimental animals, and the testing of candidate therapeutic or diagnostic agents for nephrotoxicity in laboratory animals.

A further aspect is that the method can be used to monitor patients throughout the course of an illness or at various times after a medical or surgical intervention that may alleviate the illness or carry a risk of provoking organ or tissue damage. The intervals at which samples of bodily fluids are taken for monitoring can be short, thus providing the earliest possible indication of organ damage and permitting the early institution of preventive or therapeutic measures. Monitoring the plasma or urinary concentration of individual molecular forms of NGAL for this purpose is preferably carried out at intervals not longer than 24 hours, and more preferably at shorter intervals, such as 16 hours or 12 hours, more preferably 9 hours or 6 hours, down to a suggested period of not longer than 3 hours, or even shorter, such as 15 minutes or 30 minutes or 1 hour, for instance if a potential insult is known to have occurred, e.g. during a surgical or medical procedure.

Measurement of free NGAL monomer or NGAL homodimer in a sample of bodily fluid, including but not limited to urine, plasma or serum, can be performed by any method that provides satisfactory analytical specificity, sensitivity and precision. Preferred methods are binding assays using a binding molecule specific to free NGAL monomer or a binding molecule or combination of binding molecules specific to NGAL homodimer, all said binding molecules being capable of binding to NGAL from the mammalian species from which the samples are obtained. Such binding molecules include, but are not limited to, monoclonal antibodies against NGAL or specific NGAL binding molecules generated by other means.

In a preferred method, monoclonal antibodies raised against recombinant NGAL from the mammalian species to be analyzed are used. The antibody specific to free NGAL monomer can be selected by various methods, a non-limiting example of which is described in **Example 3** below. One antibody is linked to a solid support to capture NGAL from a sample, such as a urine sample, a blood plasma or serum sample, while the other, e.g. an antibody specific to the free monomer form of NGAL, is linked to a label such as a dye complex, fluorophore, electrochemical detection moiety, biotin or enzyme, which can be detected by any of many methods known to those skilled in the art. The solid support may e.g. be a polystyrene or polyvinyl chloride surface for enzyme-linked immunosorbent assay (ELISA), latex (polystyrene) particles, paramagnetic particles, a filter frit composed of compressed polyethylene particles, or a porous nitrocellulose matrix, or indeed any suitable support used in immunochemical analyses. Particles coated with molecules that bind specifically to NGAL in the manner required by the molecular form specificity of the assay can also be used to determine the concentration of a particular molecular form of NGAL in a sample by particle-enhanced turbidimetric or nephelometric methods, whether performed manually or in automated apparatus.

A preferred means for measuring NGAL in a sample of urine or blood includes a dipstick, lateral flow (immunochromatographic) test or minicolumn test, which allows for the rapid, near-subject analysis of a sample. As will be understood by those of skill in the art upon reading this disclosure, however, other means of measuring individual molecular forms of NGAL can be used, including automated methods in central laboratories in which the apparatus permits the random access of samples for urgent analysis.

The method of the invention does not comprise a surgical, therapeutic or diagnostic step practiced on the human body.

The present disclosure covers a method of selecting a binding molecule, including a monoclonal antibody, that is capable of binding specifically to NGAL monomer by selecting said molecule from a series of candidate binding molecules according to its ability to bind to a preparation of NGAL monomer and its inability to bind to NGAL homodimer.

The following non-limiting examples are provided to further illustrate the present invention.

### EXAMPLES

### Example 1: Determination of the molecular forms of NGAL present in normal and patient samples

A volume of sample (plasma, serum, urine or recombinant human NGAL) containing a known amount of human NGAL immunoreactivity as determined by an ELISA which detects both monomer and oligomerized forms of NGAL was subjected to molecular size exclusion chromatography (gel filtration). The sample was diluted in column buffer (phosphate buffered saline: PBS, pH 7.4) and applied to a column (1.6 cm x 60 cm) of prep-grade Superdex 200 (GE Healthcare) equilibrated in the same buffer. The column was loaded and run by means of a GE Healthcare Äkta chromatographic apparatus and fractions were collected. The UV absorption of the eluate was monitored at 280 nm. Between runs, the column was washed and disinfected according to the manufacturer's instructions. NGAL immunoreactivity in the fractions was determined by means of the same ELISA. The peaks of NGAL immunoreactivity were plotted and the recovery of immunoreactivity determined.

Molecular size exclusion chromatography of plasma from a patient with ARF due to tubular necrosis is illustrated in Figure 1. UV absorption peaks corresponding to plasma macroglobulins, immunoglobulins and albumin were observed and used as approximate molecular size markers. The first peak of NGAL immunoreactivity to emerge was a very small peak emerging at the leading edge of the immunoglobulin peak. This contained higher complexed forms of NGAL including NGAL/MMP-9 complex and higher NGAL oligomers. Its content of NGAL/MMP-9 complex was confirmed by NGAL/MMP-9 ELISA (R&D Systems). This peak contained 1.0% of the total NGAL immunoreactivity that could be assigned to chromatographic peaks. A trace amount of NGAL homotrimer emerged at before and at the leading edge of the albumin peak and amounted to 0.8% of the total assignable NGAL immunoreactivity. A larger peak of NGAL immunoreactivity emerged after the albumin peak. This corresponded to NGAL homodimer and contained 22.3% of the total assignable NGAL immunoreactivity. This was followed by a very much larger peak of NGAL immunoreactivity corresponding to free NGAL monomer and containing 75.9% of the total assignable NGAL immunoreactivity. The separation of these last two peaks confirmed that the monomer and dimer forms of NGAL were not in rapid equilibrium with each other.

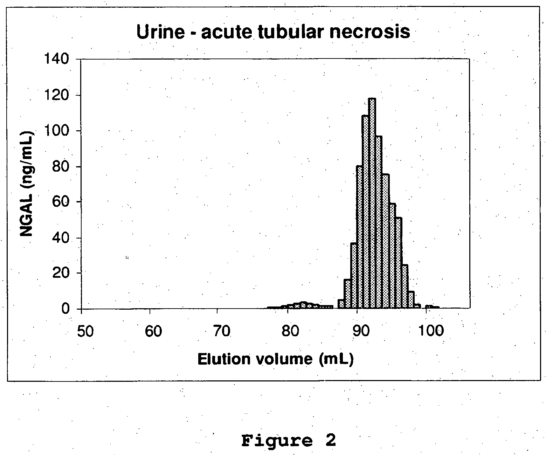

Molecular size exclusion chromatography of urine from another patient with ARF due to tubular necrosis is illustrated in Figure 2. This showed a very small peak of NGAL homodimer containing 3.0% of the total assignable NGAL immunoreactivity, followed by a very large peak of free NGAL monomer containing 97.0% of the assignable NGAL immunoreactivity. No peak corresponding to higher complexed forms of NGAL was observed.

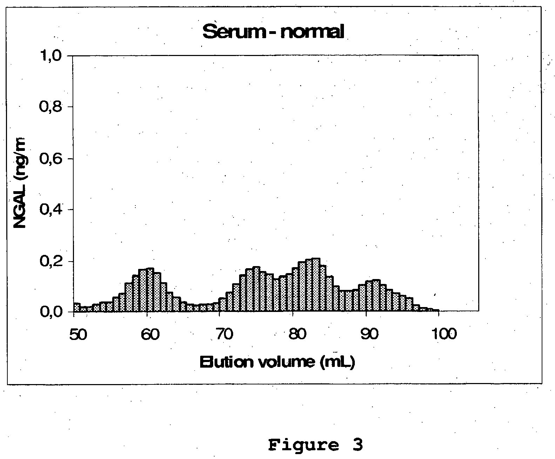

Molecular size exclusion chromatography of normal human serum (Figure 3) produced peaks of higher complexed forms of NGAL (24.1 % of assignable NGAL immunoreactivity), NGAL homotrimer (24.5% of assignable NGAL immunoreactivity), NGAL homodimer (33.1% of assignable NGAL immunoreactivity) and free NGAL monomer (18.3% of assignable NGAL immunoreactivity).

### Example 2: Preparation of human free NGAL monomers and homodimers

Native or recombinant human NGAL is isolated from respectively neutrophils or culture supernatant of prokaryotic or eukaryotic cells transformed or transfected with a vector coding for the amino-acid sequence of human NGAL, by means of protein separation techniques well known to those skilled in the art. For both native and recombinant human NGAL, the respective preparations contain both free NGAL monomer and NGAL homodimer. The monomer and homodimer forms are separated by ion-exchange chromatography, and the peaks containing respectively monomer and homodimer are identified by analyzing reduced and unreduced samples from the peaks on sodium dodecyl sulfate polyacrylamide gel electrophoresis (SDS-PAGE). Fractions containing NGAL monomer are pooled and used within 1-4 days, so that any new formation of dimer is minimal. Alternatively, the pool is treated with iodoacetamide or N-ethylmaleimide to block any free sulfhydryl groups and minimize the possibility of disulfide bridge formation. The solution is dialyzed against three changes of PBS adjusted to pH 6.5 with hydrochloric acid and stored at 4°C. Fractions containing NGAL homodimers are also pooled. The homodimer is more stable than the monomer but can, if necessary, be further stabilized by treating with a 20-fold molar excess of dithio-*bis*-(sulfosuccinimidylpropionate) (DTSSP; Pierce) as described by Yan et al. (2001), before being dialyzed and stored as above.

### Example 3: Selection of monoclonal antibodies specific for free NGAL monomer

Hybridoma supernatants derived from Balb-C mice immunized with recombinant human NGAL, principally in the monomer form, are screened by ELISA for antibody capable of binding to coated recombinant human NGAL. The antibodies from positive wells are then re-screened against i) lightly biotinylated human NGAL monomer bound to a streptavidin coat, and ii) lightly biotinylated human NGAL dimer bound to the same coat. Only those supernatants which show antibody binding to the monomer and not to the dimer are selected. It is assumed that these antibodies bind to human NGAL at an epitope which is blocked by dimer formation.

Alternatively, the immunization can be carried out with a carrier linked to a synthetic peptide with a sequence identical or closely related to that of a portion of the human NGAL protein chain, said sequence including the cysteinyl residue at position 87 of the mature protein, which is thought to participate in the formation of NGAL homodimer, followed by a similar screening procedure to that described above.

### Example 4: Sandwich ELISA specific for free NGAL monomer

The ELISA wells are coated with a monoclonal antibody to NGAL which is capable of binding both monomers and dimers. After the application of sample and washing, bound NGAL monomer is detected by means of a monoclonal antibody selected according to Example 3 to be specific for NGAL monomer and appropriately labeled to allow sensitive detection.

Alternatively, the ELISA wells are coated with a monoclonal antibody selected according to Example 3 to be specific for NGAL monomer, and bound NGAL monomer is detected by means of another, labeled, monoclonal antibody to NGAL that is capable of binding to NGAL monomer bound to the monomer-specific antibody.

The stated methods can be embodied in a kit or device for measuring free NGAL monomer, said kit or device comprising a solid support and monoclonal antibodies or other binding molecules selected according to the above principles.

### Example 5: Sandwich ELISA specific for NGAL homodimer and higher oligomers

The ELISA wells are coated with a monoclonal antibody to NGAL which is capable of binding both monomers and dimers. After the application of sample and washing, bound NGAL monomer is detected by means of the same monoclonal antibody which has been appropriately labeled to allow sensitive detection. The fact that the same antibody is used for capture and detection means that forms of NGAL will only give a signal if they contain two or more copies of the identical epitope in positions sufficiently remote from each other to allow concurrent binding of the same antibody. This makes the assay specific for homodimers and higher oligomers of NGAL.

It is not essential that the two monoclonal antibodies used be identical. It is also possible to find pairs of different antibodies whose epitopes, while not identical, are so disposed on the NGAL molecule that any binding of the second antibody to NGAL monomer is blocked by the binding of the first antibody to NGAL monomer.

The stated methods can be embodied in a kit or device for measuring NGAL homodimer and higher oligomers, said kit or device comprising a solid support and monoclonal antibodies or other binding molecules selected according to the above principles.

### Example 6: Sandwich ELISA permitting the parallel measurement of both free NGAL monomer and NGAL homodimer plus higher oligomers

The ELISA wells are coated with a monoclonal antibody to NGAL which is capable of binding both monomers and dimers. After the application of sample and washing, bound NGAL monomer is detected by means of a monoclonal antibody selected according to Example 3 to be specific for NGAL monomer and appropriately labeled to allow sensitive detection. In parallel wells to which the same sample has been applied, bound NGAL homodimer and higher oligomers are detected as in Example 5 with a labeled form of either the same antibody as that used for coating the wells or another antibody whose binding to NGAL monomer is blocked by prior binding to the coating antibody.

The stated methods can be embodied in a kit or device for the parallel measurement of both free NGAL monomer and NGAL homodimer plus higher oligomers, said kit or device comprising a solid support and monoclonal antibodies or other binding molecules selected according to the above principles.

### Example 7: Sandwich ELISA for measuring the sum of free NGAL monomer and NGAL homodimer

The reactivities of existing NGAL ELISAs with monomer, homodimer and higher homooligomer forms of NGAL have not been disclosed. Polyclonal antibodies to NGAL react with all molecular forms of NGAL, and the majority of monoclonal antibodies to NGAL also react with both monomer and dimer forms. The combinations of antibodies suitable for the specific ELISAs described in Examples 4 and 5 require special selection. It is therefore to be expected that most existing NGAL ELISAs will react to some extent with each of said molecular forms. By using the preparations of free NGAL monomers and NGAL homodimers described in Example 2, it is possible to characterize pairs of monoclonal antibodies to NGAL in terms of their quantification of these two molecular forms of NGAL as described in a) and b) below.
a) ELISA wells are coated with a first monoclonal antibody to NGAL which is capable of binding both NGAL monomers and homodimers. After the application of sample and washing, bound NGAL is detected by means of a second monoclonal antibody which is also capable of binding to NGAL monomers and homodimers and which has been appropriately labeled to allow for sensitive detection. Different pairs of first and second antibodies are tested with samples containing purified preparations of NGAL monomer and NGAL homodimer respectively, at known concentrations. Pairs of first and second antibodies are selected which give the same signal for a given mass concentration of NGAL irrespective of whether this is in the monomer or dimer form. This implies that the epitopes of such antibody pairs are so disposed on the NGAL protein chain as to permit binding of the second antibody to each NGAL subunit when NGAL monomers or dimers are bound to the first antibody. The resulting assay will give the same result for a given mass concentration of NGAL irrespective of its relative content of monomer and dimer. Ignoring the possible small contribution of higher oligomers of NGAL whose reactivity in the assay has not been quantified, a sandwich ELISA of this type can be loosely termed a "total" NGAL ELISA.
b) It is also possible to select pairs of first and second antibodies that give the same signal for an NGAL dimer molecule as for a monomer molecule. In this case, the signal obtained is not proportional to the mass concentration of NGAL but to the molar concentration of NGAL monomers and dimers. This implies that the epitopes of such antibody pairs are so disposed on the NGAL protein chain in such as way that dimerization of the NGAL blocks prevents the binding of the second antibody to both subunits of the dimer. A sandwich ELISA of this type shows a modest selectively for NGAL monomers in terms of mass concentration, as twice the mass of dimer is needed to give the same signal as a given mass of monomer.

### Example 8: NGAL dipstick test for free NGAL monomer

The analytical area of a dipstick comprised of a polystyrene surface is coated with a monoclonal capture antibody capable of binding both monomer and homodimer forms of human NGAL. An aliquot of the centrifuged, diluted sample is added to a solution of enzyme-labeled detection antibody specific for free NGAL monomer in the first tube, into which the dipstick is immersed. Complexes of enzyme-labeled detection antibody with NGAL are bound to the dipstick, which is then washed with tap water and placed in a chromogenic substrate solution in a second tube. The color developed in the substrate solution within a given time is read either by eye and compared with a chart of color intensities which indicates the concentration of NGAL in the urine sample, or in a simple colorimeter that can, for example, be programmed to indicate the NGAL concentration directly.

### Example 9: Lateral flow device for measuring free monomer NGAL

A lateral flow device comprised of a strip of porous nitrocellulose or other material with channels capable promoting the migration of liquid by capillary forces is coated near its distal end with a capture antibody capable of binding both monomer and dimer forms of NGAL, said antibody being applied as a transverse band. A further transverse band of antibody against antibodies of the species from which the detection antibody is derived is placed distally to the capture antibody band and serves as a control of strip function. The proximal end of the strip contains the detection antibody specific to free NGAL monomer adsorbed or linked to labeled polystyrene particles or particles of dye complex. When an aliquot of the centrifuged or filtered sample is applied to the proximal end of the strip, the labeled particles attached to detection antibody travel along the strip by capillary attraction. When reaching the band of capture antibody, only those particles which have bound NGAL monomer in the sample will be retained, giving rise to a detectable band. Particles reaching the control band of antibody against the detection antibody will produce a detectable band whether or not any NGAL has been bound. The intensity of the labeled bands can be read by eye in the case of colored particles or by means of the appropriate detection device for the label used. A positive result is indicated by color development or the accumulation of label in both bands, while a negative result is indicated by color development or other label only in the control band. Failure of color development or other label in the control band indicates inadequate strip function. The sensitivity of the test can be regulated by the dilution of the sample applied, which is adjusted so that only NGAL monomer concentrations above the determined cutoff values give rise to a positive result. The sensitivity of the test can also be adjusted by linking the detection antibody to a mixture of labeled and unlabeled particles. Batches of strips can be pre-calibrated and equipped with a calibration code that can be read by the detection device, so that a quantitative or semi-quantitative result can be read from the device. Many variations of the individual aspects of this lateral flow technology are possible, as known to those skilled in the art.

### Example 10: Minicolumn test for free NGAL monomer

A minicolumn contains a frit made of compressed polyethylene particles allowing the passage of fluid and cells. The frit is coated with a capture antibody against human NGAL that is capable of binding both NGAL monomers and dimers. The minicolumn is incorporated into a device, which by means of automated liquid handling allows the diluted sample to be applied at a fixed flow rate and volume, followed by a dye-complexed detection antibody that is specific to free NGAL monomer. After the passage of wash solution, the color intensity of the frit is read by light diffusion photometry. The batches of frits are pre-calibrated and the minicolumns equipped with a calibration code that can be read by the device, so that a quantitative result can be displayed by the instrument without the need for prior calibration with standards.

### Example 11: Comparison of diagnostic performance with respect to acute renal injury of measuring NGAL monomer, NGAL homodimer and "total" NGAL in plasma samples

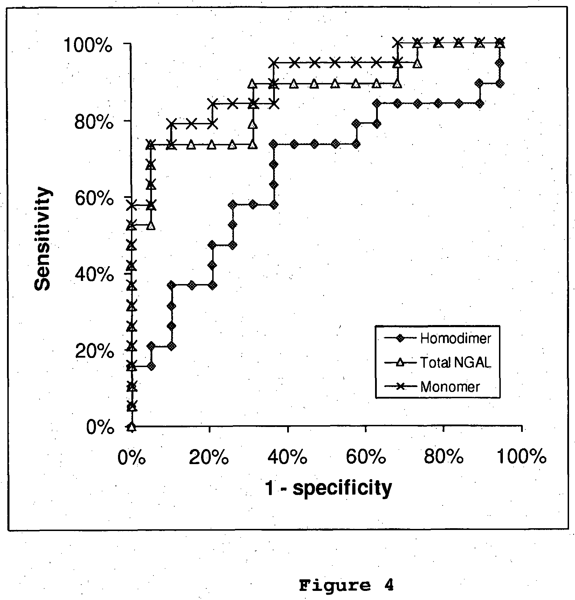

EDTA-plasma samples were selected from 38 patients admitted to an intensive care unit, 19 of whom were diagnosed clinically and biochemically as having acute renal injury, the remaining patients showing no evidence of such injury. The samples that had previously shown the highest NGAL concentration for each patient as measured according to Example 7 b) were re-assayed for NGAL homodimer according to Example 5 and for "total" NGAL according to Example 7 a), from which the concentration of NGAL monomer could also be calculated. Receiver operating characteristic (ROC) curves for the NGAL monomer, dimer and "total" values are shown in Figure 4.

The best performance with respect to the diagnosis of acute renal injury was obtained with the NGAL monomer values, the area under the curve (AUC) being 0.853 and the diagnostic sensitivity and specificity both being 84% using a cutoff value of 450 ng/mL. The diagnostic performance of "total" NGAL values was inferior (AUC 0.812, sensitivity 74% and specificity 89% at a cutoff value of 580 ng/mL). The diagnostic performance of the NGAL dimer values was very poor (AUC 0.609) to the point of not contributing to the diagnosis of acute renal injury. Furthermore, the NGAL homodimer results showed no significant correlation with the NGAL monomer results. These findings indicate that the plasma levels of NGAL monomer, but not those of NGAL homodimer, relate to the presence or absence of acute renal injury in these patients.

The cutoff value of 450 ng/mL as the plasma concentration of NGAL monomer which has to be exceeded to achieve a high diagnostic specificity for acute renal injury in this small group of patients is not necessarily representative of the cutoff values appropriate for other groups of patients. The present patient group was characterized by the occurrence in almost every patient of serious concomitant conditions known to be associated with the non-renal release of NGAL, such as severe infection including sepsis, systemic inflammation and adenocarcinomas. This raises the appropriate cutoff value for the diagnosis of acute renal injury to a high level, possibly the highest level that is likely to be found in any patient group. Other patient groups may consist of individuals who have few or no concomitant conditions that influence NGAL levels, but who may, for example, be placed at risk of acute renal injury by elective surgical interventions involving cardiopulmonary bypass. In such groups, cutoff levels for the concentration of NGAL monomer in plasma above which the concentration is diagnostic of acute renal injury may approach the upper limit of the normal range, approximately 60 ng/mL for NGAL monomer. With respect to urine concentrations, cutoff values of the NGAL monomer concentration that may be diagnostic of acute renal injury may also, for the same reason, approach the upper limit of normal, approximately 12 ng/mL.

### References

Allen RA, Erickson RW, Jesaitis AJ (1989) Identification of a human neutrophil protein of Mr 24 000 that binds N-formyl peptides: co-sedimentation with specific granules. Biochim Biophys. Acta 991:123-133.
Anonymous (2007) Product inlay of the Quantikine Human MMP-9/NGAL Complex Immunoassay kit, Catalog Number DM9L20, R&D Systems, Inc., Minneapolis, MN, USA.
Bu DX, Hemdahl AL, Gabrielsen A, Fuxe J, Zhu C, Eriksson P, Yan ZQ (2006) Induction of neutrophil gelatinase-associated lipocalin in vascular injury via activation of nuclear factor-kappaB. Am J Pathol 169:2245-2253.
Kjeldsen L, Johnsen AH, Sengelov H, Borregaard N (1993) Isolation and primary structure of NGAL, a novel protein associated with human neutrophil gelatinase. J Biol Chem 268:10425-10432.
Kjeldsen L, Koch C, Arnljots K, Borregaard N (1996) Characterization of two ELISAs for NGAL, a newly described lipocalin in human neutrophils. J Immunol Methods 198:155-164.
Liu Q, Nilsen-Hamilton M (1995) Identification of a new acute phase protein. J Biol Chem 270:22565-22570.
Monier F, Surla A, Guillot M, Morel F (2000) Gelatinase isoforms in urine from bladder cancer patients. Clin Chim Acta 299:11-23.
Venge P, Carlson M, Fredens K, Garcia R (1990) The 40 kD-protein. A new protein isolated from the secondary granules of human neutrophils. Joint International Conference on Leukocyte Biology, abstract. J Leukocyte Biol 1 (suppl.):28.
Triebel S, Blaser J, Reinke H, Tschesche H (1992) A 25 kDa alpha 2-microglobulin-related protein is a component of the 125 kDa form of human gelatinase. FEBS Lett 314:386-388.
Xu SY, Carlson M, Engstrom A, Garcia R, Peterson CG, Venge P (1994) Purification and characterization of a human neutrophil lipocalin (HNL) from the secondary granules of human neutrophils. Scand J Clin Lab Invest 54:365-376.
Yan L, Borregaard N, Kjeldsen L, Moses MA (2001) The high molecular weight urinary matrix metalloproteinase (MMP) activity is a complex of gelatinase B/MMP-9 and neutrophil gelatinase-associated lipocalin (NGAL). Modulation of MMP-9 activity by NGAL. J Biol Chem 276:37258-37265.
Zhao H, Ito A, Sakai N, Matsuzawa Y, Yamashita S, Nojima H (2006) RECS1 is a negative regulator of matrix metalloproteinase-9 production and aged RECS1 knockout mice are prone to aortic dilation. Circ J 70:615-624.

## Claims

1. A method of diagnosing, monitoring or assessing the severity of renal injury, disease or disorder by measuring the concentration of free neutrophil gelatinase-associated lipocalin (NGAL) monomer, by a method that is specific or selective for the free monomer form, in a sample of bodily fluid from the human individual and comparing said concentration with the range of concentrations of said individual molecular form that occur in individuals not known to be affected by said renal disorder or injury, whereby a deviation of the concentration from said range determines the presence of renal disorder or injury.

2. A method according to claim 1 of diagnosing, monitoring or assessing the severity of a renal injury, disease or disorder which has led to acute renal failure or which signifies an immediate risk of developing acute renal failure in a human individual, said method comprising the steps of
i) determining the concentration of free NGAL monomer in a sample of bodily fluid from the human individual and
ii) comparing said concentration value with a cutoff value determined from the range of free NGAL monomer concentrations in other human individuals which show no evidence of renal injury, disease or disorder, so that a value greater than the cutoff value indicates the presence of said renal injury, disease or disorder.

3. A method according to claim 1 of diagnosing, monitoring or determining the risk of developing acute renal failure in a human individual, wherein said method discriminates between an individual which does not have acute renal failure and is not at immediate risk of developing acute renal failure, and an individual which may have acute renal failure or is at risk of developing acute renal failure, said method comprising the steps of
i) determining the concentration of free NGAL monomer in a sample of bodily fluid from the human individual, and
ii) comparing said concentration with a predetermined cutoff value chosen so that a concentration of free NGAL monomer below the cutoff value categorizes the human individual as not having and not being at immediate risk of developing acute renal failure.

4. The method of claims 2 and 3, comprising the further step of repeating steps i) and ii) of said claims one or more times or comprising the further step of repeating steps i) and ii) of claim 2 or 3 within 24 hours, e.g. within 12 hours, such as within 6 hours, e.g. within 3 hours, such as within 1 hour, e.g. within 30 minutes or within 15 minutes or comprising the further step of repeating steps i) and ii) of claim 2 or 3 after a treatment of acute renal failure has been initiated or completed.

5. The method of any of claims 2 through 4, wherein the renal injury or the risk of developing acute renal failure is due to ischemia or is due to a complication of an inflammatory, infective or neoplastic disease or is due to critical illness of any cause requiring intensive care or is due to a surgical intervention or is due to the administration of a nephrotoxic agent.

6. The method of any of claims 1 through 5, wherein the injury is due to external physical or chemical causes or is due to exposure to radiation.

7. The method of any of the preceding claims, wherein the bodily fluid is blood plasma or blood serum or urine.

8. The method of any of the preceding claims, wherein free monomer NGAL is measured by means of a molecule that binds specifically to free NGAL monomer and not to complexed forms of NGAL in human individuals.

## Patentansprüche

1. Verfahren zur Diagnose, Überwachung und Bestimmung der Schwere einer Nieren-Verletzung, -Erkrankung oder -Störung durch Messen der Konzentration des freien Neutrophil Gelatinase-assoziierten Lipocalin Monomers (NGAL) mittels eines Verfahrens, das spezifisch oder selektiv ist für die freie Monomer-Form, in einer Probe einer Körperflüssigkeit eines menschlichen Individuums, und Vergleichen der Konzentration mit dem Bereich an Konzentrationen der individuellen Molekül-Form in Individuen, die bekanntermaßen keine Nieren-Störung oder -Verletzung aufweisen, wobei eine Abweichung der Konzentration von dem Bereich die Anwesenheit der Nieren-Störung oder -Verletzung anzeigt.

2. Verfahren nach Anspruch 1 zur Diagnose, Überwachung und Bestimmung der Schwere einer Nieren-Verletzung, -Erkrankung oder -Störung, die zu akutem Nieren-Versagen führte oder die ein unmittelbares Risiko akutes Nierenversagen zu entwickeln anzeigt, wobei das Verfahren die Schritte umfasst:
i) Bestimmen der Konzentration von freiem NGAL-Monomer in einer Probe einer Körperflüssigkeit aus dem menschlichen Individuum, und
ii) Vergleichen des Werts der Konzentration mit einem Schwellenwert, der aus einem Bereich freier NGAL-Monomer-Konzentrationen in anderen menschlichen Individuen bestimmt wurde, die kein Anzeichen von Nieren-Versagen, einer Nieren-Verletzung oder -Störung zeigen, so dass ein Wert über dem Schwellenwert die Anwesenheit des Nieren-Versagens, der Nieren-Erkrankung oder -Störung anzeigt.

3. Verfahren nach Anspruch 1 zur Diagnose, Überwachung und Bestimmung des Risikos akutes Nierenversagen zu entwickeln in einem menschlichen Individuum, wobei das Verfahren zwischen einem Individuum, das kein akutes Nieren-Versagen und kein unmittelbares Risiko akutes Nieren-Versagen zu entwickeln aufweist, und einem Individuum, das akutes Nieren-Versagen oder ein Risiko akutes Nieren-Versagen zu entwickeln aufweist, wobei das Verfahren die Schritte umfasst,
i) Bestimmen der Konzentration von freiem NGAL-Monomer in einer Probe einer Körperflüssigkeit aus dem menschlichen Individuum, und
ii) Vergleichen der Konzentration mit einem bestimmten Schwellenwert, der so gewählt ist, dass eine Konzentration an freiem NGAL-Monomer unter dem Schwellenwert kategorisiert, dass das menschliche Individuum kein akutes Nieren-Versagen und kein unmittelbares Risiko akutes Nieren-Versagen zu entwickeln aufweist.

4. Verfahren nach Anspruch 2 und 3, welches weiter den Schritt umfasst, die Schritte i) und ii) der Ansprüche ein oder zweimal zu wiederholen, oder den weiteren Schritt umfasst, die Schritte i) und ii) der Ansprüche 2 oder 3 binnen 24 Stunden, beispielsweise binnen 12 Stunden, wie binnen 6 Stunden, beispielsweise binnen 3 Stunden, wie 1 Stunde, beispielsweise binnen 30 Minuten oder binnen 15 Minuten zu wiederholen, oder den weiteren Schritt umfasst, die Schritte i) und ii) von Anspruch 2 oder 3 zu wiederholen, nachdem die Behandlung von akutem Nieren-Versagen initiiert oder beendet wurde.

5. Verfahren nach einem der Ansprüche 2 bis 4, wobei das Nieren-Versagen oder das Risiko akutes Nieren-Versagen zu entwickeln zurückzuführen ist auf eine Ischämie, oder auf eine Komplikation einer entzündlichen, infektiösen oder neoplastischen Erkrankung, oder auf eine kritische Erkrankung jeglichen Grunds, die Intensivpflege erfordert, oder auf einen chirurgischen Eingriff oder auf die Verabreichung eines nephrotoxischen Mittels.

6. Verfahren nach einem der Ansprüche 1 bis 5, wobei die Verletzung auf eine äußere körperliche oder chemische Ursache oder auf eine Aussetzung an radioaktive Strahlung zurückgeht.

7. Verfahren nach einem der vorhergehenden Ansprüche wobei die Körperflüssigkeit Blutplasma oder Blutserum oder Urin ist.

8. Verfahren nach einem der vorhergehenden Ansprüche, wobei das freie Monomer NGAL mittels eines Moleküls gemessen wird, das spezifisch an freies NGAL-Monomer bindet und nicht an komplexierte Formen von NGAL in menschlichen Individuen.

## Revendications

1. Méthode de diagnostic, surveillance ou évaluation de la gravité d'une lésion, d'une maladie ou d'un trouble rénal par mesure de la concentration d'un monomère de lipocaline neutrophile libre associée à la gélatinase (NGAL), par une méthode qui est spécifique ou sélective envers la forme monomère libre, dans un échantillon de fluide corporel provenant d'un sujet humain et comparaison de ladite concentration à la plage des concentrations de ladite forme moléculaire individuelle présentes chez des sujets non connus pour être atteints dudit trouble ou lésion rénal(e), tout écart de la concentration par rapport à ladite plage déterminant la présence d'un trouble ou d'une lésion rénal(e).

2. Méthode selon la revendication 1 de diagnostic, surveillance ou évaluation de la gravité d'une lésion, d'une maladie ou d'un trouble rénal ayant conduit à une insuffisance rénale aiguë ou synonyme de risque immédiat de développer une insuffisance rénale aiguë chez un sujet humain, ladite méthode comprenant les étapes consistant à :
i) déterminer la concentration du monomère de NGAL libre dans un échantillon de fluide corporel provenant du sujet humain, et
ii) comparer ladite valeur de concentration à une valeur de coupure déterminée à partir de la plage des concentrations du monomère de NGAL libre chez d'autres sujets humains indemnes de signes de lésion, de maladie ou de trouble rénal, de sorte qu'une valeur supérieure à la valeur de coupure indique la présence de ladite lésion, maladie ou trouble rénal.

3. Méthode selon la revendication 1 de diagnostic, surveillance ou détermination du risque de développer une insuffisance rénale aiguë chez un sujet humain, dans laquelle ladite méthode fait la distinction entre un sujet qui n'est pas atteint d'une insuffisance rénale aiguë et ne court aucun risque immédiat de développer une insuffisance rénale aiguë, et un sujet qui peut être atteint d'insuffisance rénale aiguë ou court le risque de développer une insuffisance rénale aiguë, ladite méthode comprenant les étapes consistant à
i) déterminer la concentration de monomère de NGAL libre dans un échantillon de fluide corporel provenant du sujet humain, et
ii) comparer ladite concentration à une valeur de coupure prédéterminée de façon qu'une concentration du monomère de NGAL libre inférieure à la valeur de coupure catégorise le sujet humain comme non atteint et ne courant pas de risque immédiat de développer une insuffisance rénale aiguë.

4. Méthode selon les revendications 2 et 3, comprenant l'étape supplémentaire consistant à répéter une ou plusieurs fois les étapes i) et ii) desdites revendications ou comprenant l'étape supplémentaire consistant à répéter les étapes i) et ii) de la revendication 2 ou 3 dans les 24 heures, par ex. dans les 12 heures, comme dans les 6 heures, par ex. dans les 3 heures, comme dans l'heure, par ex. dans les 30 minutes ou dans les 15 minutes qui suivent ou comprenant l'étape supplémentaire consistant à répéter les étapes i) et ii) de la revendication 2 ou 3 après qu'un traitement de l'insuffisance rénale aiguë a été démarré ou achevé.

5. Méthode selon l'une quelconque des revendications 2 à 4, dans laquelle la lésion rénale ou le risque de développer une insuffisance rénale aiguë est dû à une ischémie ou est dû à une complication d'une maladie inflammatoire, infectieuse ou néoplasique ou est dû à une maladie critique d'origine quelconque nécessitant des soins intensifs ou est dû à une intervention chirurgicale ou est dû à l'administration d'un agent néphrotoxique.

6. Méthode selon l'une quelconque des revendications 1 à 5, dans laquelle la lésion est due à des causes physiques ou chimiques externes ou est due à une exposition à un rayonnement.

7. Méthode selon l'une quelconque des revendications précédentes, dans laquelle le fluide corporel est le plasma sanguin ou le sérum sanguin ou l'urine.

8. Méthode selon l'une quelconque des revendications précédentes, dans laquelle le monomère de NGAL libre est mesuré au moyen d'une molécule qui se lie spécifiquement au monomère de NGAL libre et non à des formes complexées de la NGAL chez des sujets humains.
